(19) Europäisches Patentamt
European Patent Office
Office européen des brevets

(11) Veröffentlichungsnummer: **0 002 742**
**B1**

(12) # EUROPÄISCHE PATENTSCHRIFT

(45) Veröffentlichungstag der Patentschrift: **14.04.82**

(21) Anmeldenummer: **78101700.9**

(22) Anmeldetag: **15.12.78**

(51) Int. Cl.³: **C 07 C 15/52,** C 07 C 15/12, C 07 C 15/20, C 07 C 25/24, C 07 C 33/28, C 07 C 33/38, C 07 C 43/04, C 07 C 43/12, C 07 C 43/16, C 07 C 43/17, A 61 K 31/015

(54) **Stilbenderivate, ihre Herstellung und Arzneimittel enthaltend diese Stilbenderivate.**

(30) Priorität: **22.12.77 LU 78751**
**10.11.78 CH 11590/78**

(43) Veröffentlichungstag der Anmeldung: **11.07.79 Patentblatt 79/14**

(45) Bekanntmachung des Hinweises auf die Patenterteilung: **14.04.82 Patentblatt 82/15**

(84) Benannte Vertragsstaaten: **BE CH DE FR IT LU NL SE**

(56) Entgegenhaltungen:
**CH-A-584 670**
**DE-A-2 337 845**
**FR-A-1 576 018**
**GB-A-1 465 661**

(73) Patentinhaber: **F. HOFFMANN-LA ROCHE & CO. Aktiengesellschaft, CH-4002 Basel (CH)**

(72) Erfinder: **Loeliger, Peter, Dr., Liebrütistrasse 23, CH-4303 Kaiseraugst (CH)**

(74) Vertreter: **Lederer, Franz, Dr. et al, Patentanwälte Lederer, Meyer Lucile-Grahn-Strasse 22, D-8000 München 80 (DE)**

**EP 0 002 742 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Stilbenderivate, ihre Herstellung und Arzneimittel enthaltend diese Stilbenderivate

In der FR-PS 1 576 018 werden Stilben- und ungesättigte Naphthalinderivate erwähnt, die z.B. durch einen Phenylrest substituiert sein können. Von diesen Verbindungen ist eine pharmakologische Wirkung nicht bekannt, vielmehr werden sie als Zwischenprodukte bei der Herstellung optischer Aufheller beschrieben. In der GB-PS 1 465 661 werden Stilbenessigsäurederivate beschrieben, die anti-inflammatorisch und analgetisch wirksam sind.

Die vorliegende Erfindung betrifft neue Stilbenderivate der Formel

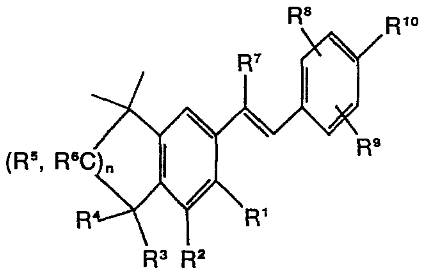

I

in der n = 1 oder 2 ist und, falls n = 1 ist, $R^1$ und $R^2$ Wasserstoff, niederes Alkoxy oder Halogen; oder, falls n = 2 ist, $R^1$ Wasserstoff, niederes Alkoxy oder Halogen und $R^2$ Wasserstoff bedeuten; $R^3$, $R^4$, $R^5$ und $R^6$ Wasserstoff oder niederes Alkyl, $R^7$ Wasserstoff, Methyl oder Äthyl, $R^8$ und $R^9$ Wasserstoff, niederes Alkyl oder Halogen darstellen und $R^{10}$ einen Rest $-(CH{=}CR^{19})_m R^{11}$ bedeutet, wobei m 0 oder 1 ist und $R^{11}$ einen Rest

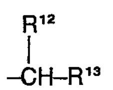 oder 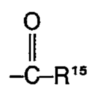

oder den 2-Oxazolinylrest, oder, falls m = 1 ist, auch Wasserstoff darstellt; $R^{12}$ Wasserstoff oder niederes Alkyl; $R^{13}$ Wasserstoff, niederes Alkyl oder einen Rest $N(R^{17}, R^{18})$ oder $-OR^{14}$, $R^{14}$ Wasserstoff, niederes Alkyl oder Alkanoyl; $R^{15}$ Wasserstoff, niederes Alkyl oder einen Rest $-OR^{16}$ oder $-(CH_2)_pN(R^{17}, R^{18})$; $R^{16}$ Wasserstoff, niederes Alkyl, Hydroxy-nieder-alkyl, Aryl, substituiertes Aryl, Aralkyl oder im Arylteil substituiertes Aralkyl; $R^{17}$ und $R^{18}$ Wasserstoff, niederes Alkyl oder gemeinsam mit dem Stickstoffatom, an das sie gebunden sind, einen heterocyclischen Rest; $R^{19}$ Wasserstoff oder niederes Alkyl und p 0, 1, 2 oder 3 bedeuten; sowie Acetale von Verbindungen der Formel I, in denen $R^{11}$ einen Rest $-C(O)R^{15}$ und $R^{15}$ Wasserstoff oder niederes Alkyl darstellt;
und Salze von Verbindungen der Formel I.

Die Erfindung betrifft weiterhin Arzneimittel auf der Basis von Verbindungen der Formel I oder deren Salze und ein Verfahren zur Herstellung der Verbindung I.

Der hier verwendete Ausdruck «nieder» bezeichnet Gruppen mit bis zu 6 Kohlenstoffatomen.

Alkyl- und Alkoxygruppen können verzweigt oder unverzweigt sein, wie beispielsweise Methyl, Äthyl, Isopropyl oder 2-Methylpropyl bzw. Methoxy, Äthoxy oder Isopropoxy.

Alkanoylgruppen leiten sich z.B. von der Essig-, Propion- oder Pivalinsäure oder auch von den höheren Carbonsäuren mit bis zu 20 Kohlenstoffatomen, z.B. von der Palmitin- oder Stearinsäure ab.

Ein bevorzugter Arylrest ist Phenyl. Beispiele von substituierten Arylresten sind Hydroxy-, Nitro- oder Halogenphenyl. Ein bevorzugter Aralkylrest ist Benzyl.

Beispiele von heterocyclischen Resten $-N(R^{17}, R^{18})$ sind 5- oder 6gliedrige stickstoffhaltige heterocyclische Reste, die ein weiteres O-, N- oder S-Atom enthalten können, z.B. Piperidino, Piperazino, Morpholino, Thiamorpholino und Pyrrolidino. Beispiele von Ketalen sind Di-nieder-alkylketale und nieder-Alkylenketale.

Der Oxazolinylrest kann durch eine oder zwei niedere Alkylgruppen substituiert sein.

Von den Halogenatomen sind Chlor und Brom bevorzugt.

Eine bevorzugte Gruppe von Verbindungen der Formel I sind diejenigen, in denen, falls n = 1 ist, $R^1$ und $R^2$ Wasserstoff, niederes Alkoxy oder Halogen, falls n = 2 ist, $R^1$ Wasserstoff, niederes Alkoxy oder Halogen, $R^2$ Wasserstoff bedeuten; $R^3$, $R^4$, $R^5$ und $R^6$ Wasserstoff oder niederes Alkyl, $R^7$ Wasserstoff, Methyl oder Äthyl, $R^8$ und $R^9$ Wasserstoff, niederes Alkyl oder Halogen darstellen, und $R^{10}$ Hydroxymethyl, Alkoxymethyl, Alkanoyloxymethyl; Carboxyl, Alkoxycarbonyl; Formyl, Alkylendioxymethyl, Alkanoyl; Carbamoyl, Mono-niederalkyl-carbamoyl, Di-niederalkylcarbamoyl, N-Heterocyclylcarbonyl oder 2-Oxazolinyl bezeichnet. Weiterhin sind die Verbindungen mit n = 2 bevorzugt, sowie diejenigen, in denen $R^1$, $R^2$, $R^5$, $R^6$, $R^8$ und $R^9$ Wasserstoff und $R^3$, $R^4$ und $R^7$ Methyl sind. Eine weitere, bevorzugte Gruppe von Verbindungen der Formel I sind diejenigen, in denen $R^{10}$ einen Rest $-(CH{=}CH)_m R^{11}$ darstellt, insbesondere jene mit m = 0 und, weiterhin, mit $R^{11}$ = nieder-Alkoxycarbonyl, nieder-Alkylcarbamoyl, nieder-Alkoxymethyl und nieder-Alkanoyloxymethyl.

Die Verbindungen der Formel I werden erfindungsgemäss dadurch erhalten, dass man eine Verbindung der allgemeinen Formel

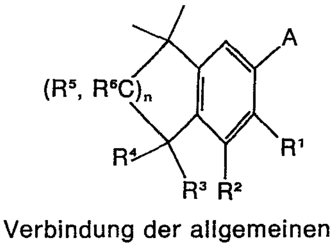

II

mit einer Verbindung der allgemeinen Formel

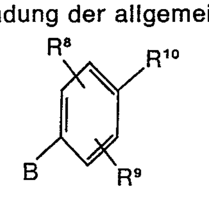

III

in denen $R^1$, $R^2$, $R^3$, $R^4$, $R^5$, $R^6$, $R^8$, $R^9$, $R^{10}$ und n die obige Bedeutung haben, und entweder A eine Triarylphosphoniumalkylgruppe der Formel $R{-}\overset{|}{C}H{-}P[Q]_3^{\oplus}\ Y^{\ominus}$ darstellt, worin R Wasserstoff, Methyl oder Äthyl, Q einen Arylrest und Y das Anion einer organischen oder anorganischen Säure bezeichnet, und B Formyl ist; oder A Formyl, Acetyl oder Propionyl ist, und B eine Dialkoxyphosphinylalkylgruppe der Formel

$$R{-}\overset{|}{C}H{-}\underset{\overset{\downarrow}{O}}{P}[Z]_2$$

darstellt, worin Z einen niederen Alkoxyrest bezeichnet; zu einer Verbindung der allgemeinen Formel I umsetzt, und erwünschtenfalls den Rest $R^{10}$ funktionell abwandelt.

Die in den genannten Triarylphosphoniumgruppen mit Q bezeichneten Arylgruppen umfassen gemeinhin alle bekannten Arylreste, insbesondere aber einkernige Reste, wie Phenyl oder nieder-Alkyl, bzw. nieder-Alkoxy-substituiertes Phenyl, wie Tolyl, Xylyl, Mesityl oder p-Methoxyphenyl.

Von den anorganischen Säureanionen Y ist das Chlor- und Brom-ion oder das Hydrosulfat-ion, von den organischen Säureanionen ist das Tosyloxy-ion bevorzugt.

Die in der Dialkoxyphosphinylalkylgruppe der Formel $R{-}\overset{|}{C}H{-}\underset{\overset{\downarrow}{O}}{P}[Z]_2$ mit Z bezeichneten Alkoxyreste sind vornehmlich niedere Alkoxyreste mit 1–6 Kohlenstoffatomen, wie Methoxy oder Äthoxy.

Die Ausgangsverbindungen der Formel II können, soweit ihre Herstellung nicht bekannt oder nachstehend beschrieben ist, in Analogie zu bekannten oder den nachstehend beschriebenen Methoden hergestellt werden.

Verbindungen der Formel II, in der A eine Formyl-, Acetyl- oder Propionylgruppe darstellt, und die Substituenten $R^1$ und $R^2$ Wasserstoff, [Oxoverbindungen der Formel II], sind, z.B. dadurch erhältlich, dass man ein der gewünschten Endverbindung der Formel I im Cyclopentenring entsprechend substituiertes Indan, oder ein im Cyclohexenring entsprechend substituiertes Tetrahydronaphthalin einer Acylierungsreaktion unterwirft. Dies kann z.B. in der Weise geschehen, dass man das Indan- bzw. Tetrahydronaphthalinderivat in Gegenwart einer Lewis-Säure acyliert.

Geeignete Acylierungsmittel sind Formaldehyd/Salzsäure Acetyl- und Propionylhalogenide z.B. Acetyl- und Propionylchlorid. Von den Lewis-Säuren sind die Halogenide von Aluminium, wie Aluminiumtrichlorid bevorzugt. Die Reaktion wird zweckmässig in einem Lösungsmittel, wie Nitrobenzol, oder in einem chlorierten Kohlenwasserstoff, wie Methylenchlorid, durchgeführt. Die Reaktionstemperatur beträgt vornehmlich 0 bis etwa +5°C.

Die erhaltene Oxoverbindung der Formel II in der $R^1$ und $R^2$ Wasserstoff bedeuten, wird erfindungsgemäss mit einem Phosphonat der Formel III, in der B eine Dialkoxyphosphinylmethylgruppe darstellt, zu Verbindungen der Formel I in der $R^1$ und $R^2$ Wasserstoff bedeuten, kondensiert.

Die für die Kondensation mit einem Aldehyd der Formel III, in der B eine Oxogruppe darstellt, benötigten Phosphoniumsalze der Formel II, in der A eine 1-(Triarylphosphonium)-methyl-[äthyl oder propyl]-gruppe bedeutet, lassen sich z.B. wie folgt herstellen:

Die vorstehend erhaltenen Oxoverbindungen der Formel II, in der $R^1$ und $R^2$ Wasserstoff bedeuten, werden bei etwa 0 bis etwa +5°C mit Hilfe eines komplexen Metallhydrids, z.B. mit Natriumborhydrid in einem Alkanol oder mit Lithiumaluminiumhydrid in einem Äther, Tetrahydrofuran oder Dioxan zu dem entsprechenden Alkohol reduziert. Der erhaltene Alkohol wird anschliessend in Gegenwart einer Aminbase, wie Pyridin mit Hilfe eines der üblichen Halogenierungsmittel, z.B. mit Phosphoroxychlorid oder Phosphortribromid halogeniert. Das erhaltene Halogenid wird danach mit einem Triarylphosphin in einem Lösungsmittel, vornehmlich mit Triphenylphosphin in Toluol oder Xylol zu dem gewünschten Phosphoniumsalz der Formel II umgesetzt.

Oxoverbindungen und Phosphoniumsalze der Formel II, in denen die Substituenten $R^1$ und $R^2$ Alkoxy bzw. Halogen sind, können z.B. dadurch hergestellt werden, dass man das entsprechende Phenol in an sich bekannter Weise durch Behandeln mit einem Alkylierungsmittel, z.B. durch Umsetzen mit einem niederen Alkylhalogenid oder mit einem niederen Alkanol in Gegenwart eines sauren Mittels, in das entsprechende Alkoxyderivat der Formel II umwandelt.

Die vorstehend genannten Phenole sind beispielsweise wie folgt zugänglich:

Man nitriert die im aromatischen Ringteil unsubstituierte Oxoverbindung der Formel II durch Behandeln mit einem Gemisch von konz. Salpetersäure und konz. Schwefelsäure. Die vorzugsweise in O-Stellung zur Formyl-, Acetyl- oder Propionylgruppe eintretende Nitrogruppe wird in an sich bekannter Weise katalytisch, z.B. mit Hilfe von Raney-Nickel, zur Aminogruppe reduziert, welche über das Diazoniumsalz in bekannter Weise in die Hydroxygruppe umgewandelt wird.

Behandelt man das aus dem Amin hergestellte Diazoniumsalz in der Wärme mit einem Kupfer(I)-halogenid, so erhält man das entsprechende Halogenderivat der Oxoverbindung der Formel II. Durch erneutes Behandeln des erhaltenen Halogenderivats mit Nitriersäure gelingt es, in m-Stellung zur Formyl-, Acetyl- oder Propionylgruppe eine Nitrogruppe einzuführen, welche ebenfalls, wie vorstehend beschrieben, in Hydroxy oder Halogen umgewandelt werden kann. Durch Umwandeln der Hydroxygruppe in Alkoxy erhält man je nach Wunsch gleich- oder gemischtsubstituierte Derivate der Ausgangsketone der Formel II.

Ein Halogenatom im aromatischen Kern kann erwünschtenfalls durch Reduktion in an sich bekannter Weise wieder entfernt werden.

Die Verbindungen der Formel III, in der B Formyl bedeutet, können aus in 1-Stellung nitrosubstituierten Phenylderivaten, wie in den Chem. Berichten 102 (1969) auf den Seiten 2502–2507 beschrieben, hergestellt werden. Sie sind auch durch Reduktion der entsprechenden p-carboxysubstituierten Phenylderivate zugänglich. Die Reduktion der Carboxyl- zur Formylgruppe kann beispielsweise mit Diisobutylaluminiumhydrid durchgeführt werden.

Die Kondensationskomponenten der Formel III, in der B eine Dialkoxyphosphinylmethylgruppe darstellt, können aus den vorstehenden Verbindungen der Formel III, in der B Formyl bedeutet in der Weise hergestellt werden, dass man die Formylgruppe mit Hilfe eines Metallhydrids, z.B. mit Hilfe von Natriumborhydrid in die Hydroxymethylgruppe umwandelt, diese mit Hilfe eines der üblichen Halogenierungsmittel, z.B. mit Phosphortrichlorid halogeniert und das erhaltene Halogenderivat mit einem Trialkylphosphit, insbesondere mit Triäthylphosphit zu dem gewünschten Phosphonat der Formel III umsetzt.

Die Kondensationskomponente der Formel III in der B Formyl oder eine Dialkoxyphosphinylmethylgruppe bedeutet, ist des weiteren auch dadurch erhältlich, dass man das entsprechende in 1-Stellung methylsubstituierte Phenylderivat halogeniert und das erhaltene Halogenmethylderivat entweder mit einem Trialkylphosphit umsetzt, oder zum Hydroxymethylderivat hydrolysiert und dieses durch Behandeln mit einem Oxydationsmittel, z.B. mit Mangandioxyd oxydiert.

Die Umsetzung der Verbindungen der Formeln II und III kann nach den bekannten Methoden der Wittig- bzw. Horner-Reaktion durchgeführt werden. Vorzugsweise setzt man als Ausgangsstoffe solche Verbindungen der Formel III ein, in denen $R^{11}$ keine gegenüber Phosphoranen reaktive Gruppe, wie insbesondere die Formylgruppe, darstellt.

Als funktionelle Abwandlung eines Substituenten $R^{10}$ in einer Verbindung der Formel I kommen z.B. in Betracht die Umwandlung einer Carboxylgruppe in ein Salz, einen Ester, ein Amid, ein Oxazolinderivat oder in die Hydroxymethylgruppe, die wiederum verestert oder veräthert werden kann; sowie die Verseifung eines Carbonsäureesters oder dessen Reduktion zur Hydroxymethylgruppe. Die Hydroxymethylgruppe kann auch zur Formylgruppe aufoxidiert werden. Verbindungen der Formel I, die einen Formylrest enthalten, können z.B. mit Hilfe einer Wittig-Reaktion zu Verbindungen der Formel I, worin $R^{10}$ einen Rest $-(CH=CR^{19})_mR^{11}$ darstellt, wobei $m = 1$, $R^{19}$ H oder Alkyl und $R^{11}$ z.B. Alkoxymethyl, Alkanoyloxymethyl, Carboxyl, Alkoxycarbonyl, Alkanyl oder Alkenyl sein kann, umgewandelt werden.

Alle diese Abwandlungen können nach an sich bekannten Methoden vorgenommen werden.

Bei der Wittig-Reaktion werden die Komponenten in Gegenwart eines säurebindenden Mittels, z.B. in Gegenwart einer starken Base, wie z.B. Butyllithium, Natriumhydrid oder dem Natriumsalz von Dimethylsulfoxyd, vornehmlich aber in Gegenwart eines gegebenenfalls durch niederes Alkyl substituierten Äthylenoxyds wie 1,2-Butylenoxyd, gegebenenfalls in einem Lösungsmittel, z.B. in einem Äther, wie Diäthyläther oder Tetrahydrofuran, oder in einem aromatischen Kohlenwasserstoff, wie Benzol in einem zwischen der Raumtemperatur und dem Siedepunkt des Reaktionsgemisches liegenden Temperaturbereich miteinander umgesetzt.

Bei der Horner-Reaktion werden die Komponenten mit Hilfe einer Base und vorzugsweise in Gegenwart eines inerten organischen Lösungsmittels, z.B. mit Hilfe von Natriumhydrid in Benzol, Toluol, Dimethylformamid, Tetrahydrofuran, Dioxan oder 1,2-Dimethoxyalkan, oder auch mit Hilfe eines Natriumalkoholats in einem Alkanol, z.B. Natriummethylat in Methanol, in einem zwischen 0° und dem Siedepunkt des Reaktionsgemisches liegenden Temperaturbereich kondensiert.

Es hat sich in bestimmten Fällen als zweckmässig erwiesen, die vorstehend genannten Reaktionen in situ, d.h. die Kondensationskomponenten ohne das betreffende Phosphoniumsalz bzw. Phosphonat zu isolieren, miteinander zu verknüpfen.

Eine Carbonsäure der Formel I kann in an sich bekannter Weise, z.B. durch Behandeln mit Thionylchlorid, vorzugsweise in Pyridin, oder Phosphortrichlorid in Toluol in das Säurechlorid übergeführt werden, das durch Umsetzen mit Alkoholen in Ester, mit Aminen in das entsprechende Amid umgewandelt werden kann. Amide können in an sich bekannter Weise in Amine, z.B. durch Reduktion mit komplexen Metallhydriden wie $LiAlH_4$, umgewandelt werden.

Ein Carbonsäureester der Formel I kann in an sich bekannter Weise, z.B. durch Behandeln mit Alkalien, insbesondere durch Behandeln mit wässriger alkoholischer Natron- oder Kalilauge in einem zwischen Raumtemperatur und dem Siedepunkt des Reaktionsgemisches liegenden Temperaturbereich hydrolysiert und entweder über ein Säurehalogenid oder, wie nachstehend beschrieben, unmittelbar amidiert werden.

Ein Carbonsäureester der Formel I kann z.B. durch Behandeln mit Lithiumamid direkt in das entsprechende Amid umgewandelt werden. Das Lithiumamid wird vorteilhaft bei Raumtemperatur mit dem betreffenden Ester zur Reaktion gebracht.

Einer Carbonsäure der Formel I kann des weiteren über das Halogenid durch Umsetzen mit 2-Aminoäthanol oder 2-Amino-2-methyl-1-propanol und anschliessende Cyclisation in ein Oxazolinderivat der Formel I umgewandelt werden.

Eine Carbonsäure oder ein Carbonsäureester der Formel I kann in an sich bekannter Weise zu dem entsprechenden Alkohol der Formel I reduziert werden. Die Reduktion wird vorteilhaft mit Hilfe eines Metallhydrids oder Alkylmetallhydrids in einem inerten Lösungsmittel durchgeführt. Als Hydride haben sich vor allem gemischte Metallhydride, wie Lithiumaluminiumhydrid oder bis-[Methoxy-äthylenoxy]-natrium-aluminiumhydrid als geeignet erwiesen. Als Lösungsmittel verwendbar sind u.a. Äther, Tetrahydrofuran oder

Dioxan, wenn Lithiumaluminiumhydrid verwendet wird; und Äther, Hexan, Benzol oder Toluol, wenn Diisobutylaluminiumhydrid oder bis-[Methoxyäthylenoxy]-natriumaluminiumhydrid eingesetzt werden.

Ein Alkohol der Formel I kann z.B. in Gegenwart einer Base, vorzugsweise in Gegenwart von Natriumhydrid, in einem organischen Lösungsmittel wie Dioxan, Tetrahydrofuran, 1,2-Dimethoxyäthan, Dimethylformamid, oder auch in Gegenwart eines Alkalimetallalkoholates in einem Alkanol, in einem zwischen 0°C und Raumtemperatur liegenden Temperaturbereich mit einem Alkylhalogenid, z.B. mit Methyljodid, veräthert werden.

Ein Alkohol der Formel I kann auch durch Behandeln mit einem Alkanoylhalogenid oder Anhydrid, zweckmässig in Gegenwart einer Base, beispielsweise in Gegenwart von Pyridin oder Triäthylamin in einem zwischen Raumtemperatur und dem Siedepunkt des Reaktionsgemisches liegenden Temperaturbereich verestert werden.

Die Carbonsäuren der Formel I bilden mit Basen, insbesondere mit den Alkalimetallhydroxyden, vorzugsweise mit Natrium- oder Kaliumhydroxyd, Salze.

Die Verbindungen der Formel I fallen mehrheitlich in der trans-Form an. Gegebenenfalls anfallende cis-Anteile können in an sich bekannter Weise, falls erwünscht, aufgetrennt oder zu den trans-Verbindungen isomerisiert werden.

Die Verfahrensprodukte der Formel I stellen pharmakodynamisch wertvolle Verbindungen dar. Sie können zur topischen und systemischen Therapie von benignen und malignen Neoplasien, von prämalignen Läsionen, sowie ferner auch zur systemischen und topischen Prophylaxe der genannten Affektion verwendet werden.

Sie sind des weiteren für die topische und systematische Therapie von Akne, Psoriasis und anderen mit einer verstärkten oder pathologisch veränderten Verhornung einhergehenden Dermatosen, wie auch von entzündlichen und allergischen dermatologischen Affektionen geeignet. Die Verfahrensprodukte der Formel I können ferner auch zur Bekämpfung von Schleimhauterkrankungen mit entzündlichen oder degenerativen bzw. metaplastischen Veränderungen eingesetzt werden.

Die neuen Verbindungen zeichnen sich gegenüber bekannten Retinoiden dadurch aus, dass sie in ausserordentlich geringen Mengen wirksam sind.

Die tumorhemmende Wirkung der Verfahrensprodukte ist signifikant. Man beobachtet im Papillomtest bei Mäusen eine Zurückbildung der mit Dimethylbenzanthracen und Krotonöl induzierten Tumoren. Die Durchmesser der Papillome nehmen im Verlauf von 2 Wochen bei intraperitonealer Applikation von p-[(E)-2-(5,6,7,8-Tetrahydro-5,5,8,8-tetramethyl-2-naphthyl)-propenyl]-benzoesäureäthylester

bei 0,2 mg/kg/Woche um 75%
bei 0,1 mg/kg/Woche um 56%
bei 0,05 mg/kg/Woche um 48%
ab.

Bei oraler Applikation von p-[(E)-2-(5,6,7,8-Tetrahydro-5,5,8,8-tetramethyl-2-naphthyl)-propenyl]-benzoesäureäthylester an Mäusen nimmt der Durchmesser der induzierten Tumoren im Verlauf von 2 Wochen [5 Einzeldosen/Woche]

bei 0,4 mg [5 × 0,08 mg]/kg/Woche um 63%
bei 0,2 mg [5 × 0,04 mg]/kg/Woche um 48%
bei 0,05 mg [5 × 0,01 mg]/kg/Woche um 37%
ab.

Die Verbindungen der Formel I und ihre Salze können ferner zur oralen Behandlung rheumatischer Erkrankungen, insbesondere solcher entzündlicher und degenerativer Art, die Gelenke, Muskeln, Sehnen und andere Teile des Bewegungsapparates befallen, verwendet werden. Beispiele solcher Erkrankungen sind die primär chronische Polyarthritis, die Spondylarthritis ankylopoetica Bechterew und die Arthropathia psoriatica.

Zur Behandlung dieser Erkrankungen werden die erfindungsgemässen Verbindungen oral, zweckmässig in einer Dosierung beim Erwachsenen von etwa 0,01–1 mg pro Tag, vorzugsweise 0,05–0,5 mg pro Tag, verabreicht. Eine etwaige Überdosierung kann sich in Form einer Vit-A-Hypervitaminose äussern, und ist an deren Symptomen (Hautschuppung, Haarausfall) leicht zu erkennen.

Die Dosis kann als Einzeldosis oder auf mehrere Teildosen verteilt verabreicht werden.

Die Verbindungen der Formel I können deshalb als Heilmittel, z.B. in Form pharmazeutischer Präparate, Anwendung finden.

Die zur systemischen Anwendung dienenden Präparate können z.B. dadurch hergestellt werden, dass man eine Verbindung der Formel I als wirksamen Bestandteil nichttoxischen, inerten, an sich in solchen Präparaten üblichen festen oder flüssigen Trägern zufügt.

Die Mittel können enteral, parenteral oder topisch verabreicht werden. Für die enterale Applikation eignen sich z.B. Mittel in Form von Tabletten, Kapseln, Dragées, Sirupen, Suspensionen, Lösungen und Suppositorien. Für die parenterale Applikation sind Mittel in Form von Infusions- oder Injektions-Lösungen geeignet.

Die Dosierungen, in denen die Verfahrensprodukte verabreicht werden, können je nach Anwendungsart und Anwendungsweg sowie nach den Bedürfnissen der Patienten variieren.

Die Verfahrensprodukte können in Mengen von etwa 0,01 bis 5 mg täglich in einer oder mehreren Dosierungen verabreicht werden. Eine bevorzugte Darreichungsform sind Kapseln mit einem Gehalt von etwa 0,1 bis 1,0 mg Wirkstoff.

Die Präparate können inerte oder auch pharmakodynamisch aktive Zusätze enthalten. Tabletten oder Granula z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde

Zusätze sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen u. dgl. bestehen. Voraussetzung ist, dass alle bei der Herstellung der Präparate verwendeten Hilfsstoffe untoxisch sind.

Zur topischen Anwendung werden die Verfahrensprodukte zweckmässig in Form von Salben, Tinkturen, Crèmen, Lösungen, Lotionen, Sprays, Suspensionen u.dgl. verwendet. Bevorzugt sind Salben und Crèmen sowie Lösungen. Diese zur topischen Anwendung bestimmten Präparate können dadurch hergestellt werden, dass man die Verfahrensprodukte als wirksamen Bestandteil nichttoxischen, inerten, für topische Behandlung geeigneten, an sich in solchen Präparaten üblichen festen oder flüssigen Trägern zumischt.

Für die topische Anwendung sind zweckmässig etwa 0,001 bis 0,3%ige, vorzugsweise 0,002 bis 0,1%ige Lösungen, sowie etwa 0,002 bis 0,5%ige, vorzugsweise etwa 0,002 bis 0,1%ige, Salben oder Crèmen geeignet.

Den Präparaten kann gegebenenfalls ein Antioxydationsmittel, z.B. Tocopherol, N-Methyl-γ-tocopheramin sowie butyliertes Hydroxyanisol oder butyliertes Hydroxytoluol beigemischt sein.

Beispiel 1

30,5 g [1-(1,1,3,3-Tetramethyl-5-indanyl)-äthyl]-triphenylphosphoniumbromid und 8 g 4-Äthoxycarbonyl-benzaldehyd werden nach Zugabe von 300 ml Butylenoxid 12 Stunden in einer Inertgasatmosphäre bei 65°C gerührt. Die entstehende klare Lösung wird gekühlt, danach in etwa 500 ml Eis/Wasser eingetragen und zweimal mit Hexan extrahiert. Der organische Extrakt wird dreimal mit Methanol/Wasser ausgeschüttelt, über Natriumsulfat getrocknet und unter vermindertem Druck eingeengt. Der Rückstand wird durch Absorption an Kieselgel [Elutionsmittel: Hexan/Äther 19 : 1] gereinigt. Der aus dem Eluat erhaltene p-[(E)-2-(1,1,3,3-Tetramethyl-5-indanyl)-propenyl]-benzoesäureäthylester schmilzt nach dem Umkristallisieren aus Äther/Hexan bei 70–71°C.

Das als Ausgangsverbindung eingesetzte [1-(1,1,3,3-Tetramethyl-5-indanyl)-äthyl]-triphenylphosphoniumbromid kann z.B. wie folgt hergestellt werden:

87,8 g Acetylchlorid werden in 240 ml Nitrobenzol gelöst. In die Lösung werden portionsweise 149,2 g Aluminiumchlorid eingetragen. Das Gemisch wird auf 0–5°C abgekühlt und hierauf unter starkem Kühlen tropfenweise mit einer Lösung von 195,0 g 1,1,3,3-Tetramethyl-indan in 360 ml Nitrobenzol versetzt. Die Temperatur soll 5°C nicht übersteigen. Das Reaktionsgemisch wird 15 Stunden bei 0°C gerührt, danach in 3 l Eis/Wasser eingetragen und mit Äther extrahiert. Der Ätherextrakt wird zweimal mit einer 2n Natriumhydroxidlösung und zweimal mit einer gesättigten Kochsalzlösung gewaschen, über Natriumsulfat getrocknet und danach zunächst im Wasserstrahlvakuum und dann, zur Entfernung des Nitrobenzols, im Hochvakuum eingeengt. Das zurückbleibende ölige (1,1,3,3-Tetramethyl-5-indanyl)-methylketon siedet bei 100–103°C/0,5 Torr.

2,66 g Lithiumaluminiumhydrid werden mit 40 ml absolutem Äther versetzt. Unter Kühlung auf 0–5°C werden innert 30 Minuten 26 g 1,1,3,3-Tetramethyl-5-indanyl-methyl-keton zugetropft. Nach weiteren 30 Minuten wird das Gemisch vorsichtig tropfenweise mit 25 ml einer gesättigten Natriumsulfatlösung versetzt. Die Reaktionslösung wird filtriert. Das Filtrat wird einmal mit einer 1n Natriumhydroxydlösung und zweimal mit einer gesättigten Kochsalzlösung gewaschen, über Natriumsulfat getrocknet und zur Entfernung des Lösungsmittels unter vermindertem Druck eingedampft. Das zurückbleibende ölige α-1,1,3,3-Pentamethyl-5-indan-methanol, eine dünnschichtchromatographisch einheitliche Verbindung [Laufmittel: Hexan/Äther 80 : 20], wird unmittelbar wie folgt weiterverarbeitet:

24,0 g α-1,1,3,3-Pentamethyl-5-indan-methanol werden in 20 ml absolutem Äther und 100 ml absolutem Hexan gelöst. Die Lösung wird nach Zugabe von 2 Tropfen Pyridin bei 0–5°C im Verlauf von 30 Minuten tropfenweise mit 16,2 g Phosphortribromid, gelöst in 80 ml absolutem Hexan, versetzt. Das Reaktionsprodukt wird nach einer weiteren Stunde Rühren bei 0–5°C in Eis/Wasser eingetragen und erschöpfend mit Äther extrahiert. Der Ätherextrakt wird je zweimal mit einer gesättigten Natriumbicarbonatlösung und Kochsalzlösung gewaschen, über Natriumsulfat getrocknet und zur Entfernung des Lösungsmittels unter vermindertem Druck eingedampft. Das zurückbleibende ölige 5-(1-Bromäthyl)-1,1,3,3-tetramethylindan, eine dünnschichtchromatographisch einheitliche Verbindung [Laufmittel: Hexan/Äther 95 : 5], wird unmittelbar wie folgt weiterverarbeitet:

26,3 g Triphenylphosphin werden in 120 ml Xylol gelöst. Die Lösung wird mit 30,9 g 5-(1-Bromäthyl)-1,1,3,3-tetramethyl-indan, gelöst in 60 ml Xylol versetzt. Das Gemisch wird unter Rühren auf 100°C erwärmt und 12 Stunden bei dieser Temperatur belassen. Das dabei anfallende, dickölige, nach Animpfen kristallisierende, 1-(1,1,3,3-Tetramethyl-5-indanyl)-äthyl-triphenylphosphoniumbromid schmilzt nach dem Umkristallisieren aus Methylenchlorid/Toluol bei 151–156°C (Kristalle enthalten 0,3 Äquivalente Toluol).

Beispiel 2

2,4 g 1,1,3,3-Tetramethyl-5-indanyl-methylketon sowie 3,4 g 4-[Diäthoxyphosphinyl)-methyl]-benzoesäureäthylester werden in 7 ml Dimethylformamid gelöst. Die Lösung wird unter Argon bei Raumtemperatur unter Rühren tropfenweise mit einer Natriumäthanolatlösung, hergestellt aus

0,33 g Natrium und 7 ml Äthanol, versetzt und anschliessend 18 Stunden bei 70°C gerührt. Das Reaktionsgemisch wird anschliessend in Eis/Wasser eingetragen und mit Äther extrahiert. Der Ätherextrakt wird mit einer gesättigten Kochsalzlösung gewaschen, über Natriumsulfat getrocknet und unter vermindertem Druck eingedampft. Der zurückbleibende p-[(E)-2-(1,1,3,3-Tetramethyl-5-indanyl)-propenyl]-benzoesäureäthylester, ein braunes Öl, wird durch Absorption an Kieselgel [Elutionsmittel: Hexan/Äther 9 : 1] gereinigt. Der Ester schmilzt nach dem Umkristallisieren aus Hexan/Äther bei 70–71°C.

Beispiel 3

In Analogie zu der in Beispiel 1 beschriebenen Arbeitsweise kann
aus [1-(5,6,7,8-Tetrahydro-5,5,8,8-tetramethyl-2-naphthyl)-äthyl]-triphenylphosphoniumbromid
und 4-Äthoxycarbonyl-benzaldehyd
der p-[(E)-2-(5,6,7-8-tetrahydro-5,5,8,8-tetramethyl-2-naphthyl)-propenyl]-benzoesäureäthylester, Smp. 90–91°C
erhalten werden.

Das als Ausgangsverbindung eingesetzte [1-(5,6,7,8-Tetrahydro-5,5,8,8-tetramethyl-2-naphthyl)-äthyl]-triphenyl-phosphoniumbromid ist in analoger Weise, wie in Beispiel 1 beschrieben,
aus 5,6,7,8-Tetrahydro-5,5,8,8-tetramethyl-naphthalin
über (5,6,7,8-Tetrahydro-5,5,8,8-tetramethyl-2-naphthyl)-methylketon
– 5,6,7,8-Tetrahydro-α-5,5,8,8-pentamethyl-2-naphthalin-methanol,
– 2-(Bromäthyl)-5,6,7,8-tetrahydro-5,5,8,8-tetramethyl-naphthalin
zugänglich.

Beispiel 4

In Analogie zu der in Beispiel 1 beschriebenen Arbeitsweise kann
aus [1-(3-Methoxy-5,6,7,8-tetrahydro-5,5,8,8-tetramethyl-2-naphthyl)-äthyl]-triphenylphosphoniumbromid
und 4-Äthoxycarbonyl-benzaldehyd
der p-[(E)-2-(3-Methoxy-5,6,7-8-tetrahydro-5,5,8,8-tetramethyl-2-naphthyl)-propenyl]-benzoesäureäthylester, Smp. 97–98°C
erhalten werden.

Das als Ausgangsverbindung eingesetzte [1-(3-Methoxy-5,6,7,8-tetrahydro-5,5,8,8-tetramethyl-2-naphthyl)-äthyl]-triphenylphosphoniumbromid ist in analoger Weise, wie in Beispiel 1 beschrieben,
aus 3-Methoxy-5,6,7,8-tetrahydro-5,5,8,8-tetramethyl-naphthalin
über 3-Methoxy-5,6,7,8-tetrahydro-5,5,8,8-tetramethyl-2-naphthyl)-methyl-keton
– 3-Methoxy-5,6,7,8-tetrahydro-5,5,8,8-tetramethyl-2-naphthalin-methanol
– 2-(1-Bromäthyl)-3-methoxy-5,6,7,8-tetrahydro-5,5,8,8-tetramethyl-naphthalin
zugänglich.

Beispiel 5

In Analogie zu der in Beispiel 1 beschriebenen Arbeitsweise kann
aus [1-(5,6,7,8-Tetrahydro-5,5,8,8-tetramethyl-2-naphthyl)-äthyl]-triphenylphosphoniumbromid
und 3-Methyl-4-äthoxycarbonyl-benzaldehyd
der p-[(E)-2-(5,6,7,8-Tetrahydro-5,5,8,8-tetramethyl-2-naphthyl)-propenyl]-2-methyl-benzoesäureäthylester als farbloses, dünnschichtchromatographisch einheitliches Öl (Rf = 0,6; Hexan/15% Äther)
erhalten werden.

Der als Kondensationskomponente eingesetzte 3-Methyl-4-äthoxycarbonyl-benzaldehyd kann ausgehend von 4-Nitro-3-methyl-benzoesäure in analoger Weise hergestellt werden wie für die Darstellung des 2-Methyl-4-äthoxycarbonyl-benzaldehydes von Huneck et al. in Chem. Ber. 102, 2502–2507 (1969) beschrieben worden ist.

Beispiel 6

In Analogie zu der in Beispiel 1 beschriebenen Arbeitsweise kann
aus [1-(1,1,2,3,3-Pentamethyl-5-indanyl)-äthyl]-triphenylphosphoniumbromid
und 4-Äthoxycarbonyl-benzaldehyd
der p-[(E)-2-(1,1,2,3,3-Pentamethyl-5-indanyl)-propenyl]-benzoesäureäthylester, Smp. 79–80°C
erhalten werden.

Beispiel 7

In Analogie zu der in Beispiel 1 beschriebenen Arbeitsweise kann
aus [1-(7-Methoxy-1,1,3,3-tetramethyl-5-indanyl)-äthyl]-triphenylphosphoniumbromid
und 4-Äthoxycarbonyl-benzaldehyd
der p-[(E)-2-(7-Methoxy-1,1,3,3-tetramethyl-5-indanyl)-propenyl]-benzoesäureäthylester, Smp. 72–73°C
erhalten werden.

Das als Ausgangsverbindung eingesetzte [1-(7-Methoxy-1,1,3,3-tetramethyl-5-indanyl)-äthyl]-triphenylphosphoniumbromid kann z. B. wie folgt hergestellt werden:

84,3 g (1,1,3,3-Tetramethyl-5-indanyl)-methylketon (Beispiel 1) werden in 160 ml konzentrierter Schwefelsäure gelöst und auf –20°C abgekühlt. Bei dieser Temperatur wird während 10 Minuten die Nitriersäure, hergestellt aus 40 ml konzentrierter Salpetersäure und 80 ml konzentrierter Schwefelsäure, zugegeben. Nach beendigter Zugabe wird der dicke Brei sofort auf Eis gegossen und zweimal mit Äther ausgezogen. Der Ätherextrakt wird mit einer Natriumbicarbonatlösung und einer Kochsalzlösung gewaschen, über Natriumsulfat getrocknet und unter vermindertem Druck vom Lösungsmittel befreit. Das anfallende (6-Nitro-1,1,3,3-tetramethyl-5-indanyl)-methylketon schmilzt nach dem Umkristallisieren aus Äther/Hexan bei 111–112°C.

75,8 g (6-Nitro-1,1,3,3-tetramethyl-5-indanyl)-methyl-keton werden in 1500 ml Methanol gelöst und unter Stickstoff mit Hilfe von 20 g Raney-Nickel 48 Stunden bei 45°C hydriert. Es werden 15 Liter Wasserstoff aufgenommen. Die Reaktionslösung wird danach durch Speedex filtriert, und das Lösungsmittel wird unter vermindertem Druck entfernt. Das anfallende (6-Aminol-1,3,3-tetramethyl-5-indanyl)-methyl-keton schmilzt nach dem Umkristallisieren aus Äther/Hexan bei 161–162°C.

113,1 g (6-Amino-1,1,3,3-tetra-methyl-5-indanyl)-methyl-keton werden in 2260 ml 20%iger Salzsäure aufgeschlämmt und auf 0–5°C abgekühlt. Das kalte Gemisch wird innerhalb 10 Minuten tropfenweise mit einer Lösung von 33,9 g Natriumnitrit in 115 ml Wasser versetzt und 30 Minuten nachgerührt. Die kalte Reaktionslösung wird anschliessend im Verlauf von 2 Stunden unter Rühren in eine Lösung von 243,2 g Kupfer(I)-chlorid in 250 ml Wasser und 250 ml konzentrierte Salzsäure, die auf 40–50° erwärmt ist, eingetropft. Das Reaktionsgemisch wird danach gekühlt, in Eiswasser eingetragen und dreimal mit Methylenchlorid ausgezogen. Der organische Extrakt wird mit einer Kochsalzlösung gewaschen, über Natriumsulfat getrocknet und unter vermindertem Druck vom Lösungsmittel befreit. Der Rückstand wird durch Adsorption an Kieselgel [Elutionsmittel Hexan/Aceton 19 : 1] gereinigt. Das aus dem Eluat erhaltene (6-Chlor-1,1,3,3-tetramethyl-5-indanyl)-methyl-keton schmilzt nach dem Umkristallisieren aus Hexan/Äther bei 69–71°C.

In Analogie zu dem vorstehend beschriebenen Verfahren können
aus (6-Chlor-1,1,3,3-tetramethyl-5-indanyl)-methyl-keton
das (6-Chlor-7-nitro-1,1,3,3-tetramethyl-5-indanyl-methyl-keton, Smp. 119–120°C;
aus (6-Chlor-7-nitro-1,1,3,3-tetramethyl-5-indanyl)-methyl-keton
das (6-Chlor-7-amino-1,1,3,3-tetramethyl-5-indanyl)-methyl-keton, Smp. 116–117°C
erhalten werden.

21,1 g (6-Chlor-7-amino-1,1,3,3-tetramethyl-5-indanyl)-methyl-keton werden in 48 ml konzentrierte Schwefelsäure eingetragen und nach dem Erwärmen auf 50°C langsam mit 140 ml destilliertem Wasser versetzt. In das Gemisch wird nach dem Abkühlen auf 0–5°C im Verlauf von 45 Minuten eine Lösung von 5,5 g Natriumnitrit in 20 ml Wasser eingetropft. Das erhaltene kalte Reaktionsgemisch wird unter Rühren im Verlauf von 2 Stunden tropfenweise in eine bei 70°C gehaltene Lösung von 60 ml Wasser und 60 ml konzentrierter Schwefelsäure eingetropft. Das Gemisch wird gekühlt, danach in Eiswasser eingetragen und dreimal mit Äther extrahiert. Die organische Phase wird mit einer Kochsalzlösung gewaschen, über Natriumsulfat getrocknet und unter vermindertem Druck vom Lösungsmittel befreit. Der Rückstand wird durch Adsorption an Kieselgel [Elutionsmittel Hexan/Äther 19 : 1] gereinigt. Das aus dem Eluat erhaltene (6-Chlor-7-hydroxy-1,1,3,3-tetramethyl-5-indanyl)-methyl-keton schmilzt nach dem Umkristallisieren aus Hexan/Äther bei 78–80°C.

4,4 g (6-Chlor-7-hydroxy-1,1,3,3-tetramethyl-5-indanyl)-methyl-keton werden in 10 ml Dimethylformamid gelöst. Die Lösung wird zuerst mit 1,1 g Kaliumhydroxid (gelöst in 1,2 ml Wasser), danach mit 5,5 ml Methyljodid versetzt und anschliessend 3 Stunden bei Raumtemperatur gerührt. Das Reaktionsgemisch wird danach in Eiswasser eingetragen und zweimal mit Äther extrahiert. Der organische Extrakt wird mehrmals mit einer Kochsalzlösung gewaschen, über Natriumsulfat getrocknet und unter vermindertem Druck vom Lösungsmittel befreit. Das anfallende (6-Chlor-7-methoxy-1,1,3,3-tetramethyl-5-indanyl)-methyl-keton schmilzt nach dem Umkristallisieren bei 59–60°C.

25 g (6-Chlor-7-methoxy-1,1,3,3-tetramethyl-5-indanyl)-methyl-keton werden in etwa 200 ml Methanol gelöst und nach Zugabe von 10 g Triäthylamin und 2,5 g 5%igem Palladium/Kohle-Katalysator bei Raumtemperatur hydriert. Im Verlauf von 5 Stunden wird ein Moläquivalent Wasserstoff aufgenommen. Die Reaktionslösung wird über Speedex filtriert. Das Filtrat wird eingedampft. Der Rückstand wird in Wasser/Äther gelöst und mehrmals mit Äther extrahiert. Der organische Extrakt wird mit Kochsalzlösung gewaschen, über Natriumsulfat getrocknet und unter vermindertem Druck vom Lösungsmittel befreit. Das anfallende (7-Methoxy-1,1,3,3-tetramethyl- 5-indanyl)-methyl-keton schmilzt nach dem Umkristallisieren aus Hexan bei 76–77°C.

In Analogie zu der in Beispiel 1 beschriebenen Arbeitsweise kann
aus (7-Methoxy-1,1,3,3-tetramethyl-5-indanyl)-methyl-keton
über 7-Methoxy-α-1,1,3,3-pentamethyl-5-indan-methanol,
– 5-(1-bromäthyl)-7-methoxy-1,1,3,3-tetramethyl-indan
das [1-(7-Methoxy-1,1,3,3-tetramethyl-5-indanyl)-äthyl]-triphenylphosphoniumbromid, Smp. 209–210°C
hergestellt werden.

Beispiel 8

In Analogie zu der in Beispiel 1 beschriebenen Arbeitsweise kann
aus [(1,1,3,3-Tetramethyl-5-indanyl)methyl]-triphenylphosphoniumchlorid
und 4-Äthoxycarbonyl-benzaldehyd
der p-[(E)-2-(1,1,3,3-tetramethyl-5-indanyl)- vinyl]-benzoesäureäthylester, Smp. 151–152°C
erhalten werden.

Das als Ausgangsverbindung eingesetzte [(1,1,3,3-Tetramethyl-5-indanyl)methyl]-triphenylphosphoniumchlorid kann z.B. wie folgt hergestellt werden:

34,2 g 1,1,3,3-Tetramethyl-indan, 150 ml Eisessig, 300 ml konz. Salzsäure und 77 ml Formaldehydlösung (35%) werden bei Raumtemperatur unter Rühren 2 Stunden auf 75–78°C erwärmt. Hierauf werden innert 10 Minuten weitere 7,7 ml 35%ige Formaldehydlösung zugetropft. Das Reaktionsgemisch wird 15 Stunden auf der gleichen Temperatur gehalten, danach gekühlt, in etwa 1 Liter Eiswasser eingetragen und erschöpfend mit Toluol extrahiert. Die organische Phase wird mit Wasser neutral gewaschen, über Natriumsulfat getrocknet und unter vermindertem Druck eingedampft. Das erhaltene Rohprodukt, ein rötliches Öl, wird über eine Vigreux-Kolonne destilliert. Das reine 5-Chlormethyl-1,1,3,3-tetramethyl-indan siedet bei 143–146°C/19 mmHg.

In Analogie zu der in Beispiel 1 beschriebenen Arbeitsweise kann
aus 5-Chlormethyl-1,1,3,3-tetramethyl-indan
und Triphenylphosphin
– [1-(1,1,3,3-Tetramethyl-5-indanyl)-methyl]-triphenyl-phosphoniumchlorid
erhalten werden.

Beispiel 9

In Analogie zu der in Beispiel 1 beschriebenen Arbeitsweise kann
aus [1-(1,1,3,3-Tetramethyl-5-indanyl)-äthyl]-triphenylphosphoniumbromid
und 4-Acetyl-benzaldehyd
das 4′-[(E)-2-(1,1,3,3-tetramethyl-5-indanyl)-propenyl]-acetophenon, Smp. 130–131°C
erhalten werden.

Beispiel 10

49 g p-[(E)-2-(5,6,7,8-tetrahydro-5-,5,8,8-tetramethyl-2-naphthyl)-propenyl]-benzoesäureäthylester (Beispiel 3) werden in 500 ml Äthanol bei 45°C gelöst und unter Rühren tropfenweise mit einer Lösung von 20 g Kaliumhydroxid in 50 ml Wasser versetzt. Das Gemisch wird 18 Stunden bei 55°C gerührt, danach gekühlt, in Eis/Wasser eingetragen, mit 3n Schwefelsäure auf pH 2 angesäuert und zweimal mit Methylenchlorid extrahiert. Der Methylenchloridextrakt wird mit einer gesättigten Kochsalzlösung gewaschen, über wasserfreiem Natriumsulfat getrocknet und unter vermindertem Druck eingeengt. Die zurückbleibende p-[(E)-2-(5,6,7,8-Tetrahydro-5,5,8,8-tetramethyl-2-naphthyl)-propenyl]-benzoesäure schmilzt nach dem Umkristallisieren aus Methylenchlorid/Hexan bei 247–248°C.

Beispiel 11

In eine Suspension von 7,0 g p-[(E)-2-(5,6,7,8-Tetrahydro-5,5,8,8-tetramethyl-2-naphthyl) propenyl]-benzoesäure (Beispiel 10) in 40 ml absolutem Äther werden nach Zugabe von 1,8 ml Pyridin unter Rühren bei 0–5°C 3,5 ml Thionylchlorid eingetropft. Die Reaktionslösung wird nach Zugabe von 5 Tropfen N,N-Dimethylformamid auf Raumtemperatur erwärmt, 18 Stunden gerührt und danach abdekantiert. Die klare gelbe Lösung des Säurechlorids wird unter Argon in eine Lösung von 3 ml Äthylamin in 20 ml absolutem Äther eingetropft. Das Gemisch wird 2 Stunden bei Raumtemperatur nachgerührt, hierauf in eine gesättigte Kochsalzlösung eingetragen und zweimal mit Äther extrahiert. Der Ätherextrakt wird mit einer gesättigten Kochsalzlösung gewaschen, über wasserfreiem Natriumsulfat getrocknet und unter vermindertem Druck eingedampft. Das zurückbleibende p-[(E)-2-(5,6,7,8-Tetrahydro-5,5,8,8-tetramethyl-2-naphthyl)-propenyl]-benzoesäuremonoäthylamid schmilzt nach dem Umkristallisieren aus Methylenchlorid/Hexan bei 177–178,5°C.

Beispiel 12

11,3 g p-[(E)-2-(5,6,7,8-Tetrahydro-5,5,8,8-tetramethyl-2-naphthyl)-propenyl]-benzoesäureäthylester (Beispiel 3) gelöst in 20 ml absolutem Äther und 20 ml absolutem Tetrahydrofuran werden bei 0–5°C in eine Aufschlämmung von 1,33 g Lithiumaluminiumhydrid in 20 ml absolutem Äther eingetropft. Die Reaktionslösung wird 12 Stunden unter Inertgas bei Raumtemperatur gerührt, danach tropfenweise bei 0–5°C mit 5 ml einer gesättigten Natriumsulfatlösung versetzt und über Speedex filtriert. Das Filtrat wird mit Äther verdünnt und einmal mit einer gesättigten Natriumbicarbonatlösung und zweimal mit einer gesättigten Kochsalzlösung gewaschen, über Natriumsulfat getrocknet und unter vermindertem Druck eingeengt. Der anfallende p-[(E)-2-(5,6,7,8-Tetrahydro-5,5,8,8-tetramethyl-2-naphthyl)-propenyl]-benzylalkohol schmilzt nach dem Umkristallisieren aus Methanol/Äther bei 123–124°C.

Beispiel 13

In eine Suspension von 6,6 g p-[(E)-2-(5,6,7,8-Tetrahydro-5,5,8,8-tetramethyl-2-naphthyl)-propenyl]-benzylalkohol (Beispiel 12) in 10 ml Äther und 10 ml Pyridin werden bei etwa 5°C unter Rühren 5,8 ml Acetylchlorid eingetropft. Das Reaktionsgemisch wird 3 Stunden bei Raumtemperatur gerührt, hierauf in etwa 100 ml Eis/Wasser eingetragen und dreimal mit Äther extrahiert. Der Ätherextrakt wird einmal mit 1n Salzsäure, danach dreimal mit einer gesättigten Kochsalzlösung gewaschen, über Natriumsulfat getrocknet und unter vermindertem Druck eingeengt. Das anfallende p-[(E)-2-(5,6,7,8-Tetrahydro-5,5,8,8-tetramethyl-2-naphthyl)-propenyl]-benzylacetat schmilzt nach dem Umkristallisieren aus Äther bei 100–101°C.

Beispiel 14

5,0 p-[(E)-2-(5,6,7,8-Tetrahydro-5,5,8,8-tetramethyl-2-naphthyl)-propenyl]-benzylalkohol (Beispiel 12), gelöst in 25 ml Dimethylformamid, werden in eine Lösung von 0,4 g Natriumhydrid in 10 ml Dimethylformamid eingetragen. Das Gemisch wird nach 1stündigem Rühren bei Raumtemperatur mit 4,3 g Methyljodid versetzt und weitere 2 Stunden gerührt. Die Reaktionslösung wird danach in etwa 200 ml Eiswasser eingetragen und dreimal mit Äther extrahiert. Der Ätherextrakt wird dreimal mit einer gesättigten Kochsalzlösung gewaschen, über Natriumsulfat getrocknet und unter vermindertem Druck eingeengt. Der anfallende p-[(E)-2-(5,6,7,8-Tetrahydro-5,5,8,8-tetramethyl-2-naphthyl)-propenyl]-benzyl-methyläther schmilzt nach dem Umkristallisieren aus Äther bei 55–56°C.

Beispiel 15

3,48 g p-[(E)-2-(5,6,7,8-Tetrahydro-5,5,8,8-tetramethyl-2-naphthyl)-propenyl]-benzoesäure (Beispiel 10) werden in 12 ml Toluol suspendiert. Die Suspension wird nach Zugabe von 3,57 g Thionylchlorid 12 Stunden bei 50°C gerührt und hierauf unter vermindertem Druck zur Trockene eingedampft. Der Rückstand wird in 6 ml Methylenchlorid gelöst. Die Lösung wird bei 0°C in eine Lösung von 2,3 g 2-Amino-2-methyl-1-propanol in 6 ml Methylenchlorid eingetropft. Die weisse Suspension wird 2½ Stunden bei Raumtemperatur gerührt, mit Essigester verdünnt, dreimal mit Wasser gewaschen, über Natriumsulfat getrocknet und unter vermindertem Druck eingeengt. Der weisse kristalline Rückstand wird in 20 ml Äther suspendiert und tropfenweise bei 0°C mit 6 g Thionylchlorid versetzt. Die weisse Suspension wird 30 Minuten bei Raumtemperatur gerührt und hierauf vorsichtig mit einer gesättigten Natriumcarbonatlösung versetzt, bis der pH-Wert ~ 9 beträgt. Die nun klare Lösung wird mit Äther verdünnt. Die Ätherphase wird dreimal mit einer gesättigten Kochsalzlösung gewaschen, über Natriumsulfat getrocknet und unter vermindertem Druck eingedampft. Das zurückbleibende 2-{p-[(E)-2-(5,6,7,8-Tetrahydro-5,5,8,8-tetramethyl-2-naphthyl)-propenyl]-phenyl}-4,4-dimethyl-2-oxazolin schmilzt nach dem Umkristallisieren aus Äther bei 115–116°C.

Beispiel 16

In Analogie zu der in Beispiel 1 beschriebenen Arbeitsweise kann
aus [1-(5,6,7,8-Tetrahydro-5,5,8,8-tetramethyl-2-naphthyl)-propyl]-triphenylphosphoniumbromid
und 4-Methoxycarbonyl-benzaldehyd
der p-[(E)-2-(5,6,7,8-Tetrahydro-5,5,8,8-tetramethyl-2-naphthyl)-1-butenyl]-benzoesäureäthylester, Smp. 82–83°C
erhalten werden.

Das als Ausgangsverbindung eingesetzte [1-(5,6,7,8-Tetrahydro-5,5,8,8-tetramethyl-2-naphthyl)propyl]- triphenylphosphoniumbromid ist in analoger Weise wie in Beispiel 1 beschrieben ausgehend von
5,6,7,8-Tetrahydro-5,5,8,8-tetramethyl-naphthalin und Propionsäurechlorid
erhältlich.

Beispiel 17

In Analogie zu der in Beispiel 11 beschriebenen Arbeitsweise kann
aus p-[(E)-2-(5,6,7,8-Tetrahydro-5,5,8,8-tetramethyl-2-naphthyl)-propenyl]-benzoesäure
und Diäthylamin
das p-[(E)-2-(5,6,7,8-Tetrahydro-5,5,8,8-tetramethyl-2-naphthyl)-propenyl]-benzoesäurediäthylamid, Smp. 111–112°C
erhalten werden.

Beispiel 18

In Analogie zu der in Beispiel 1 beschriebenen Arbeitsweise kann
aus p-[(E)-2-(5,6,7,8-Tetrahydro-5,5,8,8-tetramethyl-2-naphthyl)-propenyl]-benzoesäure
und Morpholin
das p-[(E)-2-(5,6,7,8-Tetrahydro-5,5,8,8-tetramethyl-2-naphthyl)-propenyl]-benzoesäuremorpholid, Smp. 143–144°C.
erhalten werden.

Beispiel 19

In Analogie zu der in Beispiel 11 beschriebenen Arbeitsweise kann
aus p-[(E)-2-(5,6,7,8-Tetrahydro-5,5,8,8-tetramethyl-2-naphthyl)-propenyl]-benzoesäure
und Isopropanol
der p-[(E)-2-(5,6,7,8-Tetrahydro-5,5,8,8-tetramethyl-2-naphthyl)-propenyl]-benzoesäure-isopropylester, Smp. 119–120°C
erhalten werden.

Beispiel 20

In Analogie zu der in Beispiel 11 beschriebenen Arbeitsweise kann
aus p-[(E)-2-(5,6,7,8-Tetrahydro-5,5,8,8-tetramethyl-2-naphthyl)-propenyl]-benzoesäure
und 2-Diäthylamino-äthanol
der p-[(E)-2-(5,6,7,8-Tetrahydro-5,5,8,8-tetramethyl-2-naphthyl)-propenyl]-benzoesäure-(2)-diäthylamino-äthylester, Smp. 65–66°C
erhalten werden.

Beispiel 21

6,7 g p-[(E)-2-(5,6,7,8-Tetrahydro-5,5,8,8-tetramethyl-2-naphthyl)-propenyl]-benzylalkohol (Beispiel 12) gelöst in 100 ml absolutem Äther werden innert 10 Minuten zu einer gerührten, auf 0–5°C gekühlten Aufschlämmung von Mangandioxid in 100 ml absolutem Äther getropft. Über Nacht wird bei Raumtemperatur gerührt und hierauf über Celite filtriert. Das Filtrat wird am Rotationsverdampfer zur Trockene eingeengt. Das gelbliche Öl kristallisiert. Umkristallisieren aus Äther liefert den p-[(E)-2-(5,6,7,8-Tetrahydro-5,5,8,8-tetramethyl-2-naphthyl)-propenyl]-benzaldehyd als farblose Kristalle vom Smp. 140–141°C.

Beispiel 22

In Analogie zu der in Beispiel 1 beschriebenen Arbeitsweise kann
aus [1-(5,6,7,8-Tetrahydro-5,5,8,8-tetramethyl-2-naphthyl)-äthyl]-triphenylphosphonium-bromid
und 4-Acetyl-benzaldehyd
das 4'-[(E)-2-(5,6,7,8-Tetrahydro-5,5,8,8-tetramethyl-2-methyl)-propenyl]-acetophenon, Smp. 148–149°C
erhalten werden.

Beispiel 23

3,0 g 4'-[(E)-2-(5,6,7,8-Tetrahydro-5,5,8,8-tetramethyl-2-naphthyl)-propenyl]-acetophenon (Beispiel 22) gelöst in 40 ml Benzol werden mit einer katalytischen Menge p-Toluolsulfonsäure und 0,6 g Äthylenglykol versetzt und in einer Dean-Stark-Apparatur erwärmt, wobei das gebildete Wasser laufend abgeschieden wird. Nach 2tägigem Rückflusserhitzen wird abgekühlt, in Eis/gesättigte Natriumbicarbonatlösung eingetragen und mit Äther erschöpfend extrahiert. Der Ätherextrakt wird zweimal mit einer gesättigten Kochsalzlösung gewaschen, über Natriumsulfat getrocknet und zur Entfernung des Lösungsmittels unter vermindertem Druck eingedampft. Der ölige Rückstand wird durch Absorption an Kieselgel (Elutionsmittel: Hexan/Äther 9 : 1) gereinigt. Das

aus dem Eluat erhaltene 2-Methyl-2-/p-[(E)-2-(5,6,7,8-Tetrahydro-5,5,8,8-tetramethyl-2-naphthyl)-propenyl]-phenyl/1,3-dioxolan schmilzt nach dem Umkristallisieren aus Äther bei 122–123°C.

Beispiel 24

Zu 10,4 g 4′-[(E)-2-(5,6,7,8-Tetrahydro-5,5,8,8-tetramethyl-2-naphthyl)-propenyl]-acetophenon (Beispiel 22) gelöst in 100 ml absolutem Methanol werden bei 0–5°C vorsichtig portionenweise 1,0 g Natriumborhydrid zugegeben. Die Reaktionslösung wird 1 Stunde bei 0° und 2 Stunden bei Raumtemperatur gerührt und hierauf in Eis/Wasser eingetragen und mit Äther erschöpfend extrahiert. Die Ätherlösung wird zweimal mit einer gesättigten Kochsalzlösung gewaschen, über Natriumsulfat getrocknet und unter vermindertem Druck eingeengt. Der anfallende α-Methyl-p-[(E)-2-(5,6,7,8-tetrahydro-5,5,8,8-tetramethyl-2-naphthyl)-propenyl]-benzylalkohol schmilzt nach dem Kristallisieren aus Äther bei 121–123°C.

Beispiel 25

In Analogie zu der in Beispiel 14 beschriebenen Arbeitsweise kann
aus α-Methyl-p-[(E)-2-(5,6,7,8-tetrahydro-5,5,8,8-tetramethyl-2-naphthyl)-propenyl]-benzylalkohol
das 1,2,3,4-Tetrahydro-6-[(E)-p-(1-methoxyäthyl)-α-methylstyryl]-1,1,4,4-tetramethylnaphthalin, Smp. 88–89°C
erhalten werden.

Beispiel 26

In Analogie zu der in Beispiel 13 beschriebenen Arbeitsweise kann
aus α-Methyl-p-[(E)-2-(5,6,7,8-tetrahydro-5,5,8,8-tetramethyl-2-naphthyl)-propenyl]-benzylalkohol
das α-Methyl-p-[(E)-2-(5,6,7,8-tetrahydro-5,5,8,8-tetramethyl-2-naphthyl)-propenyl]-benzylacetat, Smp. 85–86°C
erhalten werden.

Beispiel 27

In Analogie zu der in Beispiel 1 beschriebenen Arbeitsweise kann
aus [1-(5,6,7,8-Tetrahydro-5,5,8,8-tetramethyl-2-naphthyl)-äthyl]-triphenylphosphoniumbromid
und 4-Methyl-benzaldehyd
das 6-[(E)-p,α-Dimethylstyryl]-1,2,3,4-tetrahydro-1,1,4,4-tetramethylnaphthalin, Smp. 84–85°C
erhalten werden.

Beispiel 28

In Analogie zu der in Beispiel 1 beschriebenen Arbeitsweise kann
aus [1-(5,6,7,8-Tetrahydro-5,5,8,8-tetramethyl-2-naphthyl)-äthyl]-triphenylphosphoniumbromid
und 4-Isopropyl-benzaldehyd
das 6-[(E)-p-isopropyl-α-methylstyryl]-1,2,3,4-tetrahydro-1,1,4,4-tetramethylnaphthalin, Smp. 86–87°C erhalten werden.

Beispiel 29

In Analogie zu der in Beispiel 1 beschriebenen Arbeitsweise kann
aus [1-(5,6,7,8-Tetrahydro-5,5,8,8-tetramethyl-2-naphthyl)-äthyl]-triphenylphosphoniumbromid
und 2,4-Dimethyl-benzaldehyd
das 6-[(E)-α,2,4-Trimethylstyryl]-1,2,3,4-tetrahydro-1,1,4,4-tetramethylnaphthalin, Smp. 54–56°C
erhalten werden.

Beispiel 30

In Analogie zu der in Beispiel 1 beschriebenen Arbeitsweise mit jedoch vorzugsweise verlängerter Reaktionszeit kann
aus Methyl-triphenylphosphoniumbromid
und p-[(E)-2-(5,6,7,8-Tetrahydro-5,5,8,8-tetramethyl-2-naphthyl)-propenyl]-benzaldehyd (Beispiel 21)
das 1,2,3,4-Tetrahydro-1,1,4,4-tetramethyl-6-[(E)-α-methyl-p-vinylstyryl]-naphthalin, Smp. 94–95°C
erhalten werden.

Beispiel 31

In Analogie zu der in Beispiel 1 beschriebenen Arbeitsweise, mit jedoch vorzugsweise verlängerter Reaktionszeit kann
aus Äthyl-triphenylphosphoniumbromid
und p-[(E)-2-(5,6,7,8-Tetrahydro-5,5,8,8-tetramethyl-2-naphthyl)-propenyl]-benzaldehyd (Beispiel 21)
das 1,2,3,4-Tetrahydro-1,1,4,4-tetramethyl-6-[(E)-α-methyl-p-allylstyryl]-naphthalin, Smp. 64–66°C
erhalten werden.

Beispiel 32

2 g p-[(E)-2-(5,6,7,8-Tetrahydro-5,5,8,8-tetramethyl-2-naphthyl)-propenyl]-benzaldehyd (Beispiel 21) und 1,4 g Diäthylphosphonoessigsäureäthylester werden in 5 ml Dimethylformamid gelöst. Unter Rühren wird dazu bei Raumtemperatur eine Natriumalkoholatlösung (hergestellt mit 6,16 g Natrium in 3 ml absolutem Alkohol) zugegeben. Nach 18stündigem Rühren bei Raumtemperatur wird das Reaktionsgemisch auf eiskalte 1n Salzsäure gegossen und mit Äther erschöpfend extrahiert. Die Ätherphasen werden mit gesättigter Natriumbicarbonatlösung und Kochsalzlösung gewaschen und nach dem Trocknen über wasserfreiem Natriumsulfat unter vermindertem Druck eingeengt. Der Rückstand wird durch Adsorption an Kieselgel (Elutionsmittel: Hexan/Äther 19 : 1) gereinigt. Der aus dem Eluat erhaltene (E)-p-[(E)-2-(5,6,7,8-Tetrahydro-5,5,8,8-tetramethyl-2-naphthyl)-propenyl]-zimtsäureäthylester schmilzt nach dem Umkristallisieren aus Hexan/Äther bei 126–127°C.

Beispiel 33

In Analogie zu der in Beispiel 11 beschriebenen Arbeitsweise kann

aus p-[(E)-2-(5,6,7,8-Tetrahydro-5,5,8,8-tetramethyl-2-naphthyl)-propenyl]-benzoesäure und Benzylchlorid
der p-[(E)-2-(5,6,7,8-Tetrahydro-5,5,8,8-tetramethyl-2-naphthyl)-propenyl]-benzoesäurebenzylester, Smp. 113–114°C
erhalten werden.

Beispiel 34

In Analogie zu der in Beispiel 11 beschriebenen Arbeitsweise kann
aus p-[(E)-2-(5,6,7,8-Tetrahydro-5,5,8,8-tetramethyl-2-naphthyl)-propenyl]-benzoesäure und 4-Nitro-benzaldehyd
das 4-Nitrobenzyl-p-[(E)-2-(5,6,7,8-tetrahydro-5,5,8,8-tetramethyl-2-naphthyl)-propenyl]-benzoat, Smp. 183–184°C
erhalten werden.

Beispiel 35

In Analogie zu der in Beispiel 11 beschriebenen Arbeitsweise kann
aus p-[(E)-2-(5,6,7,8-Tetrahydro-5,5,8,8-tetramethyl-2-naphthyl)-propenyl]-benzoesäure und Methylenglykol
das 2-Hydroxyäthyl-p-[(E)-2-(5,6,7,8-tetrahydro-5,5,8,8-tetramethyl-2-naphthyl)-propenyl]-benbenzoat, Smp. 138–139°C
erhalten werden.

Beispiel 36

Zu einer Lösung von 14,1 g p-[(E)-2-(5,6,7,8-Tetrahydro-5,5,8,8-tetramethyl-2-naphthyl)-propenyl]-(E)-zimtsäureäthylester (Beispiel 32) in 70 ml absolutem Hexan wird bei Raumtemperatur unter einer Inertgasatmosphäre und Rühren 60 ml einer 20%igen Lösung von Dibah (Dibutylaluminiumhydrid) in Hexan zugetropft und über Nacht weitergerührt. Die Reaktionslösung wird danach tropfenweise bei 0–5°C mit 50 ml Methanol versetzt und über Speedex filtriert. Das Filtrat wird mit Äther verdünnt, einmal mit einer gesättigten Natriumbicarbonatlösung und zweimal mit einer gesättigten Kochsalzlösung gewaschen, über Natriumsulfat getrocknet und unter vermindertem Druck eingeengt. Der anfallende 3-p-/[(E)-2-(5,6,7,8-Tetrahydro-5,5,8,8-tetramethyl-2-naphthyl)-propenyl]-phenyl/-(E)-2-propen-1-ol schmilzt nach dem Umkristallisieren aus Hexan bei 109–110°C.

Beispiel 37

In Analogie zu der in Beispiel 13 beschriebenen Arbeitsweise kann ausgehend von
3-p-/[(E)-2-(5,6,7,8-Tetrahydro-5,5,8,8-tetramethyl-2-naphthyl)-propenyl]phenyl/-(E)-2-propan-1-ol
das 3-p-[(E)-2-(5,6,7,8-Tetrahydro-5,5,8,8-tetrame-thyl-2-naphthyl)-propenyl]-phenyl/-2-propen-1-yl-acetat, Smp. 109–110°C
erhalten werden.

Beispiel 38

In Analogie zu der in Beispiel 14 beschriebenen Arbeitsweise kann ausgehend von
3-p-/[(E)-2-(5,6,7,8-Tetrahydro-5,5,8,8-tetramethyl-2-naphthyl)-propenyl]-phenyl/-(E)-2-propen-1-ol
der 3-p-/[(E)-2-(5,6,7,8-Tetrahydro-5,5,8,8- tetramethyl-2-naphthyl)-propenyl]-phenyl/-2-propen-1-yl-methyläther, Smp. 88–90°C
erhalten werden.

Beispiel A

Kapseln zur oralen Verabreichung können folgende Zusammensetzung aufweisen:

| | Pro Kapsel |
|---|---|
| p-[(E)-2-(5,6,7,8-Tetrahydro-5,5,8,8-tetramethyl-2-naphthyl)-propenyl]-benzoesäureäthylester | 0,1 mg |
| Wachsmischung | 50,5 mg |
| Pflanzenöl | 98,9 mg |
| Trinatriumsalz der Äthylendiamintetraessigsäure | 0,5 mg |

Beispiel B

Eine Salbe kann folgende Zusammensetzung aufweisen:

| | | |
|---|---|---|
| p-[(E)-2-(5,6,7,8-Tetrahydro-5,5,8,8-tetramethyl-2-naphthyl)-propenyl]-benzoesäureäthylester | | 0,01 g |
| Cetylalkohol | | 2,7 g |
| Wollfett | | 6,0 g |
| Vaseline | | 15,0 g |
| Destilliertes Wasser | qu.s. ad | 100,0 g |

**Patentansprüche**

1. Verbindungen der allgemeinen Formel

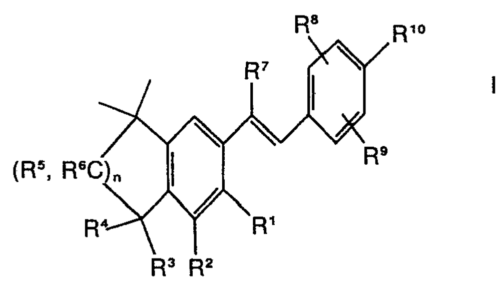

I

in der n = 1 oder 2 ist, und, falls n = 1 ist, $R^1$ und $R^2$ Wasserstoff, niederes Alkoxy oder Halogen, oder falls n = 2 ist, $R^1$ Wasserstoff, niederes Alkoxy oder Halogen und $R^2$ Wasserstoff bedeuten; $R^3$, $R^4$, $R^5$ und $R^6$ Wasserstoff oder niederes Alkyl, $R^7$ Wasserstoff, Methyl oder Äthyl, $R^8$ und $R^9$ Wasserstoff, niederes Alkyl oder Halogen darstellen und $R^{10}$ einen Rest $-(CH=CR^{19})_m R^{11}$ bedeutet, wobei m 0 oder 1 ist und $R^{11}$ einen Rest

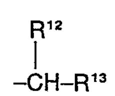 oder 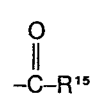

oder den 2-Oxazolinylrest, oder, falls m = 1 ist, auch Wasserstoff darstellt; $R^{12}$ Wasserstoff oder niederes Alkyl; $R^{13}$ Wasserstoff, niederes Alkyl oder einen Rest $N(R^{17}, R^{18})$ oder $-OR^{14}$,

$R^{14}$ Wasserstoff, niederes Alkyl oder Alanoyl; $R^{15}$ Wasserstoff, niederes Alkyl oder einen Rest $-OR^{16}$ oder $-(CH_2)pN(R^{17}, R^{18})$; $R^{16}$ Wasserstoff, niederes Alkyl, Hydroxy-nieder-alkyl, Aryl, substituiertes Aryl, Aralkyl oder im Arylteil substituiertes Aralkyl; $R^{17}$ und $R^{18}$ Wasserstoff, niederes Alkyl oder gemeinsam mit dem Stickstoffatom, an das sie gebunden sind, einen heterocyclischen Rest; $R^{19}$ Wasserstoff oder niederes Alkyl und p 0, 1, 2 oder 3 bedeuten; sowie Acetale von Verbindungen der Formel I,
in denen $R^{11}$ einen Rest $-C(O)R^{15}$ und $R^{15}$ Wasserstoff oder niederes Alkyl darstellt;
und Salze von Verbindungen der Formel I.

2. Verbindungen der Formel in Anspruch 1, in der, falls n = 1 ist, $R^1$ und $R^2$ Wasserstoff, niederes Alkoxy oder Halogen, falls n = 2 ist, $R^1$ Wasserstoff, niederes Alkoxy oder Halogen, $R^2$ Wasserstoff bedeuten; $R^3$, $R^4$, $R^5$ und $R^6$ Wasserstoff oder niederes Alkyl, $R^7$ Wasserstoff, Methyl oder Äthyl, $R^8$ und $R^9$ Wasserstoff, niederes Alkyl oder Halogen darstellen, und $R^{10}$ Hydroxymethyl, Alkoxymethyl, Alkanoyloxymethyl; Carboxyl, Alkoxycarbonyl; Formyl, Alkylendioxymethyl, Alkanoyl; Carbamoyl, Mono-niederalkyl-carbamoyl, Di-niederalkyl-carbamoyl, N-Heterocyclylcarbonyl oder 2-Oxazolinyl bezeichnet, und Salze davon.

3. Verbindungen der Formel in Anspruch 1, in der n = 2 ist.

4. Verbindungen gemäss Anspruch 3 und der Formel in Anspruch 1, in der $R^1$, $R^2$, $R^5$, $R^6$, $R^8$ und $R^9$ Wasserstoff; und $R^3$, $R^4$ und $R^7$ Methyl sind.

5. Verbindungen gemäss Anspruch 4 und der Formel in Anspruch 1, in der $R^{10}$ einen Rest $-(CH=CH)_mR^{11}$ darstellt.

6. Verbindungen gemäss Anspruch 5 und der Formel in Anspruch 1, in der m = 0 ist.

7. Verbindungen gemäss Anspruch 6 und der Formel in Anspruch 1, in der $R^{11}$ nieder-Alkoxycarbonyl, nieder-Alkylcarbamoyl, nieder-Alkoxymethyl oder nieder-Alkanoyloxymethyl ist.

8. p-[(E)-2-(3-Methoxy-5,6,7,8-tetrahydro-5,5,8,8-tetramethyl-2-naphthyl)-propenyl]-benzoesäureäthylester.

9. p-[(E)-2-(5,6,7,8-Tetrahydro-5,5,8,8-tetramethyl-2-naphthyl)-propenyl]-benzylacetat.

10. p-[(E)-2-(5,6,7,8-Tetrahydro-5,5,8,8-tetramethyl-2-naphthyl)-propenyl]-benzylmethyläther.

11. p-[(E)-2-(5,6,7,8-Tetrahydro-5,5,8,8-tetramethyl-2-naphthyl)-propenyl]-benzoesäuremonoäthylamid.

12. Verfahren zur Herstellung der Verbindungen der Formel I und deren Salzen, dadurch gekennzeichnet, dass man eine Verbindung der allgemeinen Formel

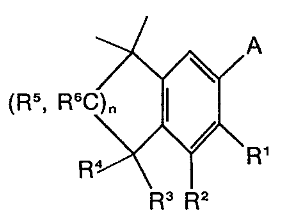
II

mit einer Verbindung der allgemeinen Formel

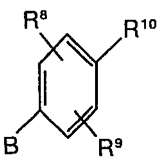
III

in denen $R^1$, $R^2$, $R^3$, $R^4$, $R^5$, $R^6$, $R^8$, $R^9$, $R^{10}$ und n die in Anspruch 1 angegebene Bedeutung haben, und entweder A eine Triarylphosphoniumalkylgruppe der Formel $R-\overset{|}{C}H-P[Q]_3^{\oplus}$ $Y^{\ominus}$ darstellt, worin R Wasserstoff, Methyl oder Äthyl, Q einen Arylrest und Y das Anion einer organischen oder anorganischen Säure bezeichnet, und B Formyl ist; oder A Formyl, Acetyl oder Propionyl ist, und B eine Dialkoxyphosphinylalkylgruppe der Formel $-R-\overset{|}{C}H-\underset{\downarrow \atop O}{P}[Z]_2$ darstellt, worin Z einen niederen Alkoxyrest bezeichnet;
zu einer Verbindung der allgemeinen Formel I umsetzt und erwünschtenfalls den Rest $R^{10}$ funktionell abwandelt.

13. Arzneimittel, enthaltend eine Verbindung der Formel I oder ein Salz davon.

14. Arzneimittel nach Anspruch 13, enthaltend p-[(E)-2-(3-Methoxy-5,6,7,8-tetrahydro-5,5,8,8-tetramethyl-2-naphthyl)-propenyl]-benzoesäureäthylester.

15. Arzneimittel nach Anspruch 13, enthaltend p-[(E)-2-(5,6,7,8-Tetrahydro-5,5,8,8-tetramethyl-2-naphthyl)-propenyl]-benzylmethyläther.

## Claims

1. Compounds of the general formula

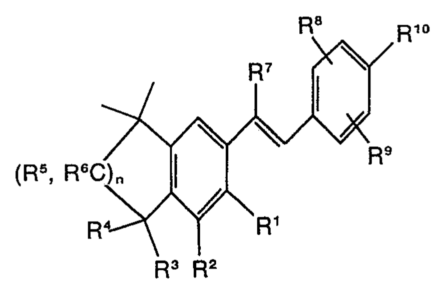
I

in which n is = 1 or 2 and, when n is = 1, $R^1$ and $R^2$ signify hydrogen, lower alkoxy or halogen, or, when n is = 2, $R^1$ signifies hydrogen, lower alkoxy or halogen and $R^2$ signifies hydrogen; $R^3$, $R^4$, $R^5$ and $R^6$ represent hydrogen or lower alkyl, $R^7$ represents hydrogen, methyl or ethyl, $R^8$ and $R^9$ represent hydrogen, lower alkyl or halogen and $R^{10}$ signifies a group $-(CH=CR^{19})_mR^{11}$, whereby m is 0 or 1 and $R^{11}$ represents a group

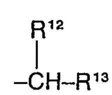 oder 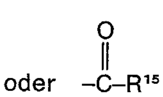

or the 2-oxazolinyl group, or, when m is = 1, also represents hydrogen; $R^{12}$ signifies hydrogen or lower alkyl; $R^{13}$ signifies hydrogen, lower alkyl or

a group $N(R^{17}, R^{18})$ or $-OR^{14}$, $R^{14}$ signifies hydrogen, lower alkyl or alkanoyl; $R^{15}$ signifies hydrogen, lower alkyl or a group $-OR^{16}$ or $-(CH_2)pN(R^{17}, R^{18})$; $R^{16}$ signifies hydrogen, lower alkyl, hydroxy-lower-alkyl, aryl, substituted aryl, arylalkyl or arylalkyl substituted in the aryl portion; $R^{17}$ and $R^{18}$ signify hydrogen, lower alkyl or together with the nitrogen atom to which they are attached a heterocyclic group; $R^{19}$ signifies hydrogen or lower alkyl and p signifies 0, 1, 2 or 3; as well as acetals of compounds of formula I in which $R^{11}$ represents a group $-C(O)R^{15}$ and $R^{15}$ represents hydrogen or lower alkyl;
and salts of compounds of formula I.

2. Compounds of the formula in claim 1, in which $R^{10}$ signifies hydroxymethyl, alkoxymethyl, alkanoyloxymethyl; carboxyl, alkoxycarbonyl; formyl, alkylenedioxymethyl, alkanoyl; carbamoyl, mono-lower alkyl-carbamoyl, di-lower alkyl-carbamoyl, N-heterocyclylcarbonyl or 2-oxazolinyl, and salts thereof.

3. Compounds of the formula in claim 1, in which n is $=2$.

4. Compounds in accordance with claim 3 and the formula in claim 1, in which $R^1$, $R^2$, $R^5$, $R^6$, $R^8$ and $R^9$ are hydrogen; and $R^3$, $R^4$ and $R^7$ are metyhl.

5. Compounds in accordance with claim 4 and the formula in claim 1, in which $R^{10}$ represents a group $-(CH=CH)_mR^{11}$.

6. Compounds in accordance with claim 5 and the formula in claim 1, in which m is $=0$.

7. Compounds in accordance with claim 6 and the formula in claim 1, in which $R^{11}$ is lower-alkoxycarbonyl, lower-alkylcarbamoyl, lower-alkoxymethyl or lower-alkanoyloxymethyl.

8. p-[(E)-2-(3-Methoxy-5,6,7,8-tetrahydro-5,5,8,8-tetramethyl-2-naphthyl)-propenyl]-benzoic acid ethyl ester.

9. p-[(E)-2-(5,6,7,8-Tetrahydro-5,5,8,8-tetramethyl-2-naphthyl)-propenyl]-benzyl acetate.

10. p-[(E)-2-(5,6,7,8-Tetrahydro-5,5,8,8-tetramethyl-2-naphthyl)propenyl]-benzyl methyl ether.

11. p-[(E)-2-(5,6,7,8-Tetrahydro-5,5,8,8-tetramethyl-2-naphthyl)propenyl]-benzoic acid monoethylamide.

12. A process for the manufacture of the compounds of formula I and their salts, characterized by reacting a compound of the general formula

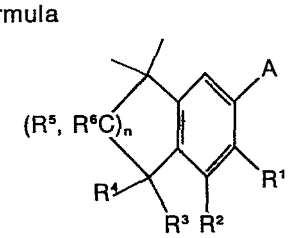

II

with a compound of the general formula

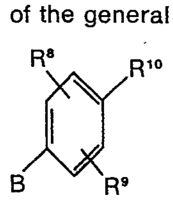

III

in which $R^1$, $R^2$, $R^3$, $R^4$, $R^5$, $R^6$, $R^8$, $R^9$, $R^{10}$ and n have the significance given in claim 1, and either A represents a triarylphosphoniumalkyl group of the formula $R-CH-P[Q]_3^{\oplus}\ Y^{\ominus}$, wherein R signifies hydrogen, methyl or ethyl, Q signifies an aryl group and Y signifies the anion of an organic or inorganic acid, and B is formyl; or A is formyl, acetyl or propionyl and B represents a dialkoxyphosphinylalkyl group of the formula $R-CH-P[Q]_3^{\oplus}\ Y^{\ominus}$, wherein Z signifies a lower alkoxy group;
to a compound of general formula I and, if desired, functionally modifying the group $R^{10}$.

13. A medicament containing a compound of formula I or a salt thereof.

14. A medicament according to claim 13 containing p-[(E)-2-(3-methoxy-5,6,7,8-tetrahydro-5,5,8,8-tetramethyl-2-naphthyl)-propenyl]-benzoic acid ethyl ester.

15. A medicament according to claim 13 containing p-[(E)-2-(5,6,7,8-tetrahydro-5,5,8,8-tetramethyl-2-naphthyl)-propenyl]-benzyl methyl ether.

**Revendications**

1. Composés de formule générale I

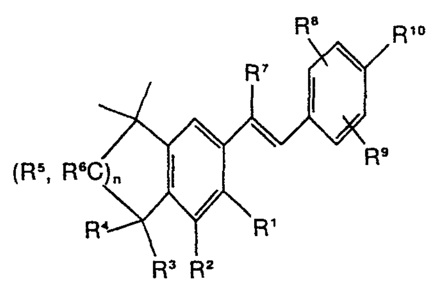

I

dans laquelle n est égale à 1 ou 2 et, si n est égal à 1, $R^1$ et $R^2$ représentent de l'hydrogène, un groupe alcoxy inférieur ou un halogène ou, si n est égal à 2, $R^1$ représente de l'hydrogène, un groupe alcoxy inférieur ou un halogène et $R^2$ représente de l'hydrogène, cependant que dans l'un ou l'autre cas, $R^3$, $R^4$, $R^5$ et $R^6$ représentent de l'hydrogène ou un groupe alkyle inférieur, $R^7$ représente de l'hydrogène ou un groupe méthyle ou éthyle, $R^8$ et $R^9$ représentent de l'hydrogène, un groupe alkyle inférieur ou un halogène, $R^{10}$ représente un reste $-(CH=CR^{19})_mR^{11}$ dans lequel m est égal à zéro ou 1 et $R^{11}$ représente un reste

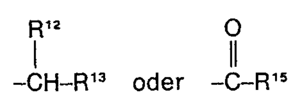

ou le reste 2-oxazolinyle ou aussi bien, si $m=1$, de l'hydrogène, $R^{12}$ représente de l'hydrogène ou un groupe alkyle inférieur, $R^{13}$ représente de l'hydrogène, un groupe alkyle inférieur ou un reste $-N(R^{17}, R^{18})$ ou $-OR^{14}$, $R^{14}$ représente de l'hydrogène, un groupe alkyle infèrieur ou un

groupe alcanoyle inférieur, $R^{15}$ représente de l'hydrogène, un groupe alkyle inférieur ou un reste $-OR^{16}$ ou $-(CH_2)pN(R^{17}, R^{18})$, $R^{16}$ représente de l'hydrogène, un groupe alkyle inférieur, un groupe hydroxyalkyle inférieur, un groupe aryle substitué ou non ou un groupe aralkyle substitué ou non dans la partie aryle, $R^{17}$ et $R^{18}$ représentent de l'hydrogène ou un groupe alkyle inférieur ou, conjointement avec l'atome d'azote auquel ils sont fixés, un reste hétérocyclique, $R^{19}$ représente de l'hydrogène ou un groupe alkyle inférieur et p est égal à zéro, 1, 2 ou 3, ainsi que les acétals de composés de formule I dans lesquels $R^{11}$ représente un reste $-C(O)R^{15}$ et $R^{15}$ de l'hydrogène ou un groupe alkyle inférieur, ainsi que les sels de ces composés.

2. Composés de formule I selon la revendication 1, dans laquelle $R^{10}$ représente un groupe hydroxyméthyle, alcoxyméthyle, alcanoyloxyméthyle, carboxyle, alcoxycarbonyle, formyle, alkylènedioxyméthyle, alcanoyle, carbamoyle, monoalkylcarbamoyle inférieur, dialkylcarbamoyle à restes alkyle inférieurs, N-hétérocyclylcarbonyle ou 2-oxazolinyle, et leurs sels.

3. Composés de formule I selon la revendication 1, dans laquelle n est égal à 2.

4. Composés de formule I selon la revendication 3, dans laquelle $R^1$, $R^2$, $R^5$, $R^6$, $R^8$ et $R^9$ représentent de l'hydrogène tandis que $R^3$, $R^4$ et $R^7$ représentent un groupe méthyle.

5. Composés de formule I selon la revendication 4, dans laquelle $R^{10}$ représente un reste $-(CH=CH)_mR^{11}$.

6. Composés de formule I selon la revendication 5, dans laquelle m est égal à zéro.

7. Composés de formule I selon la revendication 6, dans laquelle $R^{11}$ représente un groupe alcoxycarbonyle inférieur, un groupe alkylcarbamoyle inférieur, un groupe alcoxyméthyle inférieur ou un groupe alcanoyloxyméthyle inférieur.

8. Le p-[(E)-2-(3-méthoxy-5,6,7,8-tétrahydro-5,5,8,8-tétraméthyl-2-naphtyl)-propényl]-benzoate d'éthyle.

9. L'acétate de p-[(E)-2-(5,6,7,8-tétrahydro-5,5,8,8-tétraméthyl-2-naphtyl)-propényl]- benzyle.

10. L'éther de p-[(E)-2-(5,6,7,8-tétrahydro-5,5,8,8-tétraméthyl-2-naphtyl)-propényl]-benzyle et de méthyle.

11. Le monoéthylamide de l'acide p-[(E)-2-(5,6,7,8-tétrahydro-5,5,8,8-tétraméthyl-2-naphtyl)-propényl]-benzoïque.

12. Procédé pour la préparation des composés de formule I selon la revendication 1 et de leurs sels, caractérisé en ce que l'on fait réagir un composé de formule générale

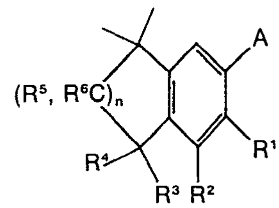

II

avec un composé de formule générale

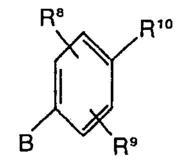

III

formules dans lesquelles $R^1$, $R^2$, $R^3$, $R^4$, $R^5$, $R^6$, $R^8$, $R^9$, $R^{10}$ et n ont les significations données à propos de la revendication 1 et soit que A représente un groupe triarylphosphoniumalkyle de formule:

$$R-CH-P(Q)_3^{\oplus} \quad Y^{\ominus}$$

dans laquelle R représente de l'hydrogène ou un groupe méthyle ou éthyle, Q représente un reste aryle et Y représente l'anion d'un acide organique ou minéral, B représentant le reste formyle, soit que A représente un reste formyle, acétyle ou propionyle, B représentant un groupe dialcoxyphosphinylalkyle de formule:

$$R-CH-\underset{\underset{O}{\|}}{P}(Z)_2$$

dans laquelle Z représente un groupe alcoxy inférieur, pour former un composé de formule générale I et, si on le désire on modifie fonctionnellement le reste $R^{10}$.

13. Médicament caractérisé par le fait qu'il contient un composé de formule I ou l'un de ses sels.

14. Médicament selon la revendication 13, caractérisé par le fait qu'il contient du p-[(E)-2-(3-méthoxy-5,6,7,8-tétrahydro-5,5,8,8-tétraméthyl-2-naphthyl)-propényl]-benzoate d'éthyle.

15. Médicament selon la revendication 13, caractérisé par le fait qu'il contient de l'éther de p-[(E)-2-(5,6,7,8-tétrahydro-5,5,8,8-tétraméthyl-2-naphtyl)-propényl]-benzyle et de méthyle.